# FASCICULE DE BREVET EUROPEEN

(11) **EP 1 446 422 B1**
(45) Date de publication et mention de la délivrance du brevet: **31.08.2011**
(21) Numéro de dépôt: 02795382.7
(22) Date de dépôt: 18.11.2002
(51) Int. Cl.: C07K 14/35

(54) **ANTIGENE MYCOBACTERIEN RECOMBINANT DE TYPE HEMAGGLUTININE DE LIAISON A L'HEPARINE (HBHA) METHYLEE**
METHYLIERTES REKOMBINANTES MYKOBAKTERIELLES ANTIGEN, DASS EINEM HEPARIN-BINDENDEN HÄMAGGLUTININ (HBHA) ENTSPRICHT
METHYLATED HEPARIN-BINDING HEMAGGLUTININ RECOMBINANT MYCOBACTERIAL ANTIGEN, PREPARATION METHOD AND IMMUNOGENIC COMPOSITIONS COMPRISING SAME

(30) Priorité: 19.11.2001 FR 0114953
(43) Date de publication de la demande: 18.08.2004
(60) Demande divisionnaire: 10183249.1
(73) Titulaire: INSTITUT PASTEUR DE LILLE, 59019 Lille cedex (FR); INSTITUT NATIONAL DE LA SANTE ET DE LA RECHERCHE MEDICALE (INSERM), 75654 Paris Cédex 13 (FR)
(72) Inventeur: PETHE, Kévin, Singapore 138 685 (SG); MENOZZI, Franco, B-7022 Hyon (BE); LOCHT, Camille, B-1180 Bruxelles (BE)
(74) Mandataire: Desaix, Anne
(86) Numéro de dépôt international: PCT/FR2002/003942
(87) Numéro de publication internationale: WO 2003/044048

(56) Documents cités:
- US-A- 5 717 064
- PETHE ET AL: "Characterization of the heparin-binding site of the mycobacterial heparin-binding hemagglutinin adhesin" JOURNAL OF BIOLOGICAL CHEMISTRY, vol. 275, 12 mai 2000 (2000-05-12), pages 14273-14280, XP002209336
- JOYS ET AL: "The susceptibility to tryptic hydrolysis of peptide bonds involving epsilon-N-methyllysine" BIOCHIMICA ET BIOPHYSICA ACTA, no. 581, 1979, pages 360-362, XP001040367 ISSN: 0006-3002
- PETHE ET AL: "Functional and immunological characterizations of the lysine-rich repeats in the C-terminal domain of the mycobacterial heparin-binding hemagglutinin" ABSTRACTS OF THE GENERAL MEETING OF THE AMERICAN SOCIETY FOR MICROBIOLOGY, THE SOCIETY, WASHINGTON, DC, US, vol. 99, no. 231/U-106, 30 mai 1999 (1999-05-30), page 654 XP000930090 ISSN: 1060-2011
- PONCZ ET AL: "The resistance to tryptic hydrolysis of peptide bonds adjacent to Ne,N-dimethyllysyl residues" JOURNAL OF BIOLOGICAL CHEMISTRY, vol. 258, 10 février 1983 (1983-02-10), pages 1844-1850, XP002242136
- PETHE: "Mycobacterial heparin-binding hemagglutinin and laminin-binding protein share antigenic methyllysines that confer resistance to proteolysis" PROCEEDINGS OF THE NATIONAL ACADEMY OF SCIENCES, USA, vol. 99, 6 août 2002 (2002-08-06), pages 10759-10764, XP002242098

## Description

La présente invention s'inscrit dans le cadre de la recherche et de la mise au point de nouveaux vaccins pour le traitement des infections mycobactériennes, notamment la tuberculose.

L'invention concerne une séquence peptidique recombinante immunogène méthylée, correspondant à l'hémagglutinine de liaison à l'héparine (HBHA) identifiée chez des souches mycobactériennes telles que *Mycobacterium tuberculosis* et *M*. *bovis. BCG* (Menozzi et coll. 1996. J. Exp. Med. 184 : 993-1001).

L'invention concerne également des procédés de préparation d'une séquence peptidique immunogène comprenant la HBHA recombinante, ladite séquence étant méthylée par modification post-traductionnelle. L'invention concerne en particulier des procédés de méthylation chimique ou enzymatique d'une séquence peptidique comprenant la HBHA et préalablement produite sous une forme recombinante non méthylée.

L'invention concerne en outre des outils, vecteurs et hôtes cellulaires recombinants, pour la mise en oeuvre des procédés chimiques ou enzymatiques de méthylation post-traductionnelle de la HBHA recombinante.

L'invention concerne enfin des compositions immunogènes comprenant la HBHA méthylée, native ou recombinante, de telles compositions étant utiles pour la préparation de vaccins contre les infections mycobactériennes.

Les mycobactéries sont des bacilles dont l'habitat est très diversifié. Selon les espèces, ces bactéries peuvent coloniser le sol, l'eau, les plantes, les animaux et/ou l'homme. Certaines espèces, telles que *M*. *smegmatis,* sont des saprophytes non pathogènes. D'autres espèces, en revanche, sont des pathogènes plus ou moins sévères des animaux et/ou de l'homme. Ainsi, *M. avium* provoque des infections chez l'oiseau. *M. bovis* est responsable de la tuberculose bovine, bien qu'elle ait été également mise en cause dans des cas de tuberculose humaine. Chez l'homme, la tuberculose est principalement causée par l'espèce hautement pathogène *M*. *tuberculosis. M. leprae* est quant à elle responsable de la lèpre, une autre pathologie de l'homme qui sévit essentiellement dans les pays en voie de développement.

La tuberculose demeure à l'heure actuelle un problème de santé publique préoccupant, dans la mesure où elle représente la première cause de mortalité liée à un agent infectieux unique. L'Organisation Mondiale de la Santé (OMS) a recensé 8,8 millions de cas de tuberculose en 1995 (Dolin et coll. 1994. Bull. WHO 72 : 213-220). Plus récemment, l'OMS a publié des chiffres alarmants, avec 10 millions de nouveaux cas de tuberculose par an et une mortalité frappant 3 millions de personnes par an (Dye et coll. 1999. J. Am. Med. Assoc. 282 : 677-686). On estime qu'un tiers de la population mondiale est infecté par *M. tuberculosis.* Cependant, toutes les personnes infectées ne développent pas la maladie.

Le problème posé par la tuberculose s'est aggravé dans les années 80, avec l'émergence et le développement de la pandémie due au syndrome d'immunodéficience acquise (SIDA). Ainsi, le nombre de cas de tuberculose associée à l'immunodépression provoquée par le rétrovirus VIH, responsable du SIDA, n'a cessé d'augmenter.

Pour être efficace, le traitement médicamenteux de la tuberculose exige généralement d'être prolongé, surtout chez les patients également infectés par le virus VIH. Dans le passé, les infections à *M. tuberculosis* étaient efficacement enrayées par certains antibiotiques, parmi lesquels la rifampicine, l'isoniazide et le pyrazinamide. Cependant, les antibiothérapies ont rapidement montré des limites dans le traitement curatif de la tuberculose du fait, d'une part, de l'émergence de souches de *M. tuberculosis* résistantes aux antibiotiques, en particulier à l'isoniazide, et d'autre part, de la toxicité de certaines molécules anti-tuberculeuses, dont le pyrazinamide.

Un seul vaccin est autorisé et couramment utilisé depuis plus de 75 ans pour prévenir l'infection tuberculeuse. Il s'agit du bacille de Calmette et Guérin, dit vaccin BCG. Ce vaccin consiste en une forme vivante d'une souche de *M. bovis,* isolée en 1908 chez une vache, et rendue avirulente *in vitro* afin d'en permettre l'administration par voie parontérale chez l'homme. Cependant, ce vaccin est actuellement sujet à controverse en ce qu'il présente des limites, notamment en terme d'efficacité. En effet, selon de nombreux essais cliniques effectués de par le monade, l'efficacité de protection obtenue grâce au vaccin BCG varie de 0 à 85 % (Fine, P.E. 1989. Rev. Infect. Dis. 11 Suppl. 2: S353-S359). Une méta-analyse suggère que l'efficacité moyenne du BCG n'excéderait pas 50 % de protection vis-à-vis de la tuberculose pulmonaire (Colditz et coll. 1994. Jama 271 : 698-702). De plus, le vaccin BCG s'avère relativement efficace chez l'enfant, tandis que son effet protecteur est quasiment nul chez l'adulte. En outre, dans la mesure où le vaccin BCG consiste en une souche mycobactérienne vivante, son administration n'est pas dénuée d'effets secondaires sur l'organisme humain, et ce, bien qu'il s'agisse d'une souche atténuée. De tels effets secondaires se manifestant a fortiori chez les personnes immunodéprimées, la vaccination de ces patients par le BCG est en conséquence à proscrire. Ce problème ne peut être contourné en tuant et inactivant les BCG étant donné qu'en pareil cas, ils perdent toute activité protectrice (Orme, I.M. 1988. Infect. Immun. 56 : 3310-3312).

La présente invention a donc pour but de remédier aux inconvénients du vaccin BCG, en proposant une nouvelle composition immunogène susceptible d'être utilisée comme vaccin contre la tuberculose. Cette composition immunogène peut également être utilisée de manière plus générale, dans le cadre de la prévention des infections mycobactériennes.

La tuberculose est une maladie de contact, qui se transmet par voie aérienne. Une fois inhalés, les germes de *M. tuberculosis* parviennent jusqu'aux poumons, qui constituent le foyer infectieux initial. A partir des poumons, les germes sont rapidement disséminés par voie sanguine ou lymphatique vers d'autres régions de l'organisme.

La séquence entière du génome de la souche de *M. tuberculosis* la mieux caractérisée à ce jour, à savoir H37Rv, a été déterminée et analysée afin d'approfondir les connaissances relatives à la biologie de ce pathogène, et d'identifier de nouvelles cibles permettant de mettre au point de nouveaux traitements thérapeutiques, c'est-à-dire prophylactiques ou curatifs (Cole et coll. 1998. Nature 393 : 537-544). Ainsi, l'approche actuelle consiste à créer des banques génomiques à partir de l'ADN de *M. tuberculosis* et à cribler lesdites banques pour identifier de nouvelles cibles thérapeutiques potentielles. De manière intéressante, il a été observé que les souches de *M. tuberculosis* présentent entre elles une grande homogénéité génétique, les changements de nucléotides d'une séquence génomique à l'autre étant très rares. Aussi, la plupart des protéines sont identiques à travers toutes les souches de cette espèce. Ceci s'avère particulièrement important au niveau de l'immunité et du développement de vaccins, dans la mesure où les marqueurs antigéniques à cibler sont quasiment ubiquitaires.

En dépit de l'importante incidence des infections mycobactériennes, l'on connaît encore peu de choses sur les mécanismes moléculaires primaires impliqués dans leur pathogénèse.

L'un des événements majeurs de la pathogénèse de la tuberculose est l'adhérence des microorganismes aux cellules cibles. Les macrophages alvéolaires ont longtemps été considérés comme la porte d'entrée de *M. tuberculosis,* et sont supposés transporter les bactéries des poumons vers les autres organes. Cependant, il a récemment été montré que *M. tuberculosis* était également capable d'interagir avec les cellules épithéliales, parmi lesquelles les cellules M, ce qui pourrait permettre aux bacilles de traverser directement la barrière épithéliale (Teitelbaum et coll. 1999. Immunity 10: 641-650). La contribution relative de chacun de ces mécanismes, ainsi que les facteurs bactériens impliqués dans la dissémination extra-pulmonaire de *M. tuberculosis* restent à ce jour inconnus.

Les souches de *M. tuberculosis* produisent à leur surface une adhésine appelée HBHA pour heparin-binding hemagglutinin adhesin (Menozzi et coll. 1996. J. Exp. Med. 184 : 993-1001). Cette protéine est également produite par d'autres mycobactéries pathogènes, telles que *M. leprae* et *M. avium* (Reddy et coll. 2000. J. Infect. Dis. 181 : 1189-1193). En revanche, la HBHA n'est pas produite par l'espèce saprophyte non pathogène *M. smegmatis* (Pethe et coll. 2001. Mol. Microbiol. 39 : 89-99).

La liaison de *M. tuberculosis* aux cellules épithéliales est inhibée par des anticorps anti-HBHA ou par compétition avec l'héparine. Ceci n'étant pas le cas avec les macrophages, cette observation suggère que l'adhérence conférée par la HBHA est spécifique des cellules non phagocytaires. Le mécanisme de cette adhérence repose sur la reconnaissance par le domaine carboxy-terminal riche en résidus lysines de la HBHA (Pethe et coll. 2000. J. Biol. Chem. 275 : 14273-14280), de récepteurs contenant des glycosaminoglycanes sulfatés portés par les cellules épithéliales.

Plus récemment, des travaux ont montré que la HBHA ne jouait pas de rôle prépondérant dans les étapes initiales de l'infection tuberculeuse, pas plus que dans la persistance des mycobactéries au niveau des poumons (Pethe et coll. 2001. Nature 412 : 190-194). Ces chercheurs ont également montré que la HBHA n'était pas requise pour la colonisation et la survie dans la rate. En revanche, la HBHA joue un rôle crucial dans la dissémination extra-pulmonaire des mycobactéries. Cette adhésine est en conséquence un facteur de virulence dont la liaison aux cellules non-phagocytaires représente une étape essentielle du processus de dissémination des mycobactéries des poumons vers la rate et potentiellement d'autres organes tels que le foie, les os, les reins ou, éventuellement, le cerveau.

La présente invention a pour but d'utiliser, dans le cadre d'un traitement essentiellement prophylactique, le pouvoir antigénique de la HBHA, dont le rôle est primordial dans la dissémination des microorganismes chez les sujets infectés.

Le clonage du gène codant la HBHA et son expression chez *Escherichia coli* ont suggéré que la protéine subit une modification post-traductionnelle (Menozzi et coll. 1998. Proc. Natl. Acad. Sci. USA 95 : 12625-12630). Dans cette publication, les auteurs avaient émis l'hypothèse que la HBHA native pouvait être glycosylée, hypothèse qui s'est par la suite révélée inexacte. Les travaux les plus récents montrent en effet que la seule modification post-traductionnelle covalente que subit la HBHA produite par *M. tuberculosis* est une méthylation complexe des résidus lysines contenus dans le domaine carboxy-terminal de la protéine.

Dans le cadre de la présente invention, les inventeurs révèlent la nature de la modification post-traductionnelle portée par la HBHA native, à savoir une méthylation covalente complexe, ladite modification lui conférant un pouvoir antigénique protecteur contre des infections à mycobactéries. La séquence peptidique de la HBHA recombinante, produite après expression de son gène dans *E. coli* par exemple, ne présente aucune activité protectrice, et ce, tant qu'elle ne subit pas de modification post-traductionnelle à l'instar de la HBHA native.

L'invention concerne donc une séquence peptidique recombinante immunogène comprenant un antigène méthylé correspondant à la HBHA native ou à la partie C-terminale de celle-ci.

Au sens de la présente invention, le terme « séquence peptidique » désigne tout ou partie de la séquence de la protéine HBHA, dès lors que ladite « séquence peptidique » contient au moins le domaine carboxy-terminal riche en résidus lysines qui assure la liaison à l'héparine. La séquence dudit domaine carboxy-terminal est la suivante :
KKAAPAKKAAPAKKAAPAKKAAAKKAPAKKAAAKKVTQK

Cette séquence a été divulguée dans la demande internationale publiée sous le numéro WO 97/44463.

L'on entend par « protéine », « protéine HBHA » ou « HBHA » au sens de la présente invention, tout ou partie de la séquence peptidique de la HBHA, à la condition toutefois d'y inclure au moins le domaine C-terminal de ladite HBHA. Lorsque la séquence considérée comprend au plus le domaine C-terminal de la HBHA, on parle avantageusement de « peptide ». On désigne notamment par « peptides » les produits issus d'une digestion enzymatique de la HBHA. Cependant, l'utilisation du terme « peptide » n'est pas limitée à ce cas d'espèce, « peptide » pouvant également être synonyme de « protéine » dans le cadre de l'invention.

Une séquence peptidique « recombinante » selon l'invention correspond à une séquence peptidique obtenue par expression, chez un hôte cellulaire hétérologue, d'une séquence nucléotidique codant ladite séquence peptidique. En particulier, ledit hôte cellulaire hétérologue peut être une bactérie n'appartenant pas au genre *Mycobacterium,* par exemple *E. coli,* ou bien d'autres organismes tels que les levures et les cellules animales et végétales.

L'expression « séquence nucléotidique » désigne toute séquence d'ADN codant une séquence peptidique telle que définie dans le cadre de la présente invention.

Conformément à l'acception usuelle, un « antigène » désigne toute séquence peptidique selon la présente invention ayant un pouvoir immunogène. En particulier, un antigène conforme à l'invention pourra être restreint au domaine de liaison à l'héparine carboxy-terminal de la HBHA.

Dans le contexte de l'invention, les expressions « domaine de liaison à l'héparine carboxy-terminal », « domaine de liaison à l'héparine », « domaine carboxy-terminal » et « domaine C-terminal » de la HBHA désignent la même région de ladite HBHA, région dont la séquence est fournie supra. Ces expressions sont donc équivalentes.

De façon préférée, la séquence peptidique recombinante immunogène selon la présente invention est méthylée au niveau du domaine de liaison à l'héparine de la HBHA. En particulier, les groupements méthyles sont portés par des résidus lysines présents dans ledit domaine de liaison à l'héparine.

Dans un mode de réalisation encore préféré de la présente invention, les groupements méthyles sont portés par tous ou partie seulement des résidus lysines présents dans le domaine C-terminal de la HBHA, sous réserve que la séquence peptidique ainsi méthylée présente une activité immunogène.

Avantageusement, au moins treize résidus lysines sur les quinze présents dans le domaine C-terminal sont méthylés. Les deux résidus lysines non méthylés sont notamment les acides aminés distaux de la séquence indiquée supra du domaine C-terminal de la HBHA.

Les résidus lysines méthylés sont préférentiellement mono- ou diméthylés.

Dans l'article de Menozzi et coll., 1998, supra, il a également été montré que la HBHA native était reconnue par deux anticorps monoclonaux, à savoir 3921 E4 et 4057D2 (Rouse et coll. 1991. Infect. Immun. 59 : 2595-2600), tandis que la forme recombinante de la HBHA non modifiée de manière post-traductionnelle n'était pas reconnue par l'anticorps 4057D2, indiquant qu'un des épitopes de la HBHA native était absent de la HBHA recombinante.

La séquence peptidique recombinante immunogène selon la présente invention, à savoir la forme recombinante de la HBHA méthylée de façon post-traductionnelle, est reconnue par l'anticorps monoclonal 4057D2, et ce, contrairement à la forme recombinante non méthylée de ladite HBHA, comme décrit dans les exemples qui suivent.

L'invention concerne également des procédés de préparation d'une séquence peptidique immunogène comprenant la HBHA recombinante, ladite séquence étant méthylée par modification post-traductionnelle.

En particulier, un procédé de préparation selon la présente invention comprend au moins les étapes suivantes :
- a): la production de la protéine HBHA recombinante chez un hôte cellulaire hétérologue - cette forme de la HBHA étant non méthylée -
- b): la purification de ladite protéine par des méthodes conventionnelles ; et
- c): la méthylation post-traductionnelle de la HBHA recombinante purifiée.

Il est entendu que, dans le cadre de l'invention, l'étape de purification de la protéine HBHA peut être réalisée avant ou, selon un autre mode de réalisation, après l'étape de méthylation de ladite protéine.

Le procédé de préparation selon l'invention permet d'obtenir la protéine HBHA recombinante méthylée ou, alternativement, tout peptide méthylé comprenant au moins le domaine de liaison à l'héparine de ladite protéine. En particulier, ledit peptide méthylé obtenu par le procédé selon l'invention correspond au dit domaine de liaison à l'héparine de la HBHA.

Avantageusement, l'hôte cellulaire hétérologue utilisé dans le cadre du procédé de préparation selon l'invention est une bactérie, notamment *E. coli* ou *M. smegmatis.* En particulier, l'hôte utilisé est *M. smegmatis.*

Les méthodes de purification de protéine sont connues de l'homme du métier et ne font pas, en tant que telles, l'objet de la présente invention. Par exemple, les propriétés de liaison à l'héparine conférées par le domaine C-terminal de la HBHA peuvent être mises à profit en pratiquant une purification de ladite HBHA par affinité sur colonne héparine-sépharose (Pethe et coll., 2000, supra).

L'invention concerne en particulier des procédés de méthylation chimique et enzymatique d'une séquence peptidique comprenant la HBHA préalablement produite sous une forme recombinante non méthylée.

Par « production sous une forme recombinante », l'on entend la production du peptide par expression dans un hôte hétérologue procaryote ou eucaryote quel qu'il soit. La production peut être obtenue à partir d'une culture cellulaire ou *in vivo* comme, par exemple, dans le lait ou dans une plante.

La méthylation chimique selon l'invention est inspirée de la littérature (Means, G.E. 1977. Meth. Enzymol. 47: 469-478). En particulier, la réaction de méthylation chimique est effectuée dans une solution comprenant du formaldéhyde et du NaBH₄.

Les procédés de méthylation enzymatique selon l'invention peuvent être mis en oeuvre à l'aide d'une ou plusieurs méthyltranférases mycobactériennes. Lesdites méthyltranférases catalysent le transfert de groupements méthyles d'un donneur vers un accepteur, en l'espèce la séquence peptidique de la HBHA recombinante préalablement purifiée. Le donneur de radical méthyle peut être notamment la S-adénosylméthionine (AdoMet), bien connue de l'homme du métier.

Plus particulièrement, la ou les méthyltransférases sont présentes et actives dans les extraits de protéines totales de mycobactéries telles que *M. bovis* BCG et *M. smegmatis*.

la ou les méthyltranférases mycobactériennes peuvent être purifiées à partir des extraits protéiques totaux de souches de mycobactéries, avant d'être placées dans le milieu réactionnel afin de catalyser la ou les réactions de transméthylation du donneur vers l'accepteur.

Des vecteurs et hôtes cellulaires recombinants peuvent être utilisés pour la mise en oeuvre des procédés enzymatiques de méthylation post-traductionnelle de la HBHA recombinante.

En particulier, un hôte cellulaire recombinant est capable de coexprimer les séquences nucléotidiques codant la HBHA et la ou les méthyltranférases mycobactériennes. Ledit hôte cellulaire est de préférence une bactérie, et en particulier une souche de *E. coli.*

Le terme « coexprimer » définit, dans le contexte de l'invention, la faculté, pour un hôte cellulaire donné, d'exprimer au moins deux séquences nucléotidiques distinctes.

L'hôte cellulaire est caractérisé par le fait qu'il peut héberger simultanément au moins deux vecteurs recombinants, vecteurs parmi lesquels l'un code la HBHA tandis que le ou les autres codent la ou les méthyltranférases mycobactériennes.

En particulier, l'hôte cellulaire peut héberger autant de vecteurs recombinants que de protéines différentes à produire, chaque vecteur codant alors une protéine mycobactérienne recombinante distincte.

Les termes « vecteur », « vecteur d'expression » et « plasmide » sont utilisés dans le contexte de la présente invention pour désigner de manière indifférente le même outil de clonage et d'expression de séquences nucléotiques, classique pour l'homme du métier.

Toutes les protéines mycobactériennes recombinantes, ou seulement partie d'entre elles, sont codées par le même vecteur d'expression.

En particulier, l'hôte cellulaire peut héberger un seul vecteur d'expression à partir duquel sont produites toutes les protéines mycobactériennes, à savoir la HBHA et la ou les méthyltransférases.

Lorsque l'hôte cellulaire héberge un seul vecteur, la production de chaque protéine mycobactérienne, HBHA ou méthyltranférase, est contrôlée par des séquences de régulation distinctes ou, selon un autre mode de réalisation, par les mêmes séquences de régulation.

En particulier, la production de tout ou partie des protéines recombinantes est contrôlée par les mêmes séquences de régulation.

Un vecteur d'expression peut coder avantageusement la HBHA et au moins une méthyltransférase mycobactérienne.

Alternativement, un vecteur d'expression code une seule protéine mycobactérienne recombinante choisie parmi la HBHA et la ou les méthyltransférases.

La présente invention concerne non seulement les hôtes cellulaires et les vecteurs d'expression, tels que définis supra, pour la mise en oeuvre des procédés de méthylation enzymatique selon l'invention.

La présente invention porte en outre sur un procédé d'obtention d'une séquence peptidique immunogène comprenant la HBHA recombinante, ladite séquence étant méthylée par modification post-traductionnelle, ledit procédé comprenant au moins les étapes suivantes :
a) la co-production de la protéine HBHA et de la ou des méthyltransférases mycobactériennes par un hôte cellulaire tel que défini précédemment ;
b) la méthylation post-traductionnelle de la HBHA recombinante par la ou les méthyltranférases recombinantes ; et
c) la purification de la HBHA recombinante méthylée par des méthodes conventionnelles.

L'invention concerne en outre des séquences peptidiques recombinantes immunogènes méthylées susceptibles d'être obtenues *in vivo* par un procédé enzymatique, ou *in vitro* par un procédé chimique ou enzymatique.

L'invention concerne enfin des compositions immunogènes comprenant la HBHA méthylée, native ou recombinante, de telles compositions étant utiles pour la préparation de vaccins contre les infections mycobactériennes.

En particulier, une composition immunogène selon la présente invention comprend, dans une formulation pharmaceutiquement acceptable, un principe actif qui est une séquence peptidique méthylée choisie parmi la séquence peptidique de la HBHA native et la séquence peptidique de la HBHA recombinante.

Une « formulation pharmaceutiquement acceptable » correspond, au sens de l'invention, à une formulation médicamenteuse susceptible d'être utilisée chez l'homme, à des doses acceptables *in vivo* eu égard à la toxicité et la pharmacologie des composés concernés, tout en étant efficaces sur le plan thérapeutique, et en particulier du point de vue immunogène.

Dans un mode de réalisation préféré de l'invention, la séquence peptidique méthylée servant de principe actif est associée à un ou plusieurs adjuvants.

Par « adjuvant » ou « composé adjuvant », l'on entend au sens de la présente invention, un composé apte à induire ou à augmenter la réponse immunitaire spécifique vis-à-vis d'un antigène ou immunogène, ladite réponse consistant indifféremment en une réponse humorale et/ou cellulaire. Cette réponse immunitaire passe généralement par une stimulation de la synthèse d'immunoglobulines spécifiques d'un antigène donné, en particulier des IgG, IgA et IgM, ou de cytokines.

Le principe actif, séquence peptidique méthylée de la HBHA, ainsi que le ou les adjuvants, sont généralement mélangés à des excipients pharmaceutiquement compatibles, tels que l'eau, un tampon salin, du dextrose, du glycérol, de l'éthanol, ou des mélanges de ceux-ci.

De telles compositions immunogènes sont préparées sous la forme de solutions liquides, ou de suspensions injectables, ou encore sous une forme solide, par exemple lyophilisée, adaptée à la mise en solution préalablement à l'injection.

Une composition immunogène selon la présente invention est formulée de manière à permettre une administration par des voies aussi diverses que la voie nasale, orale, sous-cutanée, intradermique, intramusculaire, vaginale, rectale, oculaire, ou auriculaire. En particulier, le choix des composés auxiliaires est dicté par le mode d'administration choisi. De tels composés auxiliaires peuvent être notamment des agents mouillants, émulsifiants ou tampons.

Avantageusement, une composition immunogène selon l'invention comprend, par dose, de 0,1 à 20 µg, et de préférence 5 µg, de protéine HBHA purifiée.

La présente invention est illustrée, sans pour autant être limitée, par les figures suivantes :

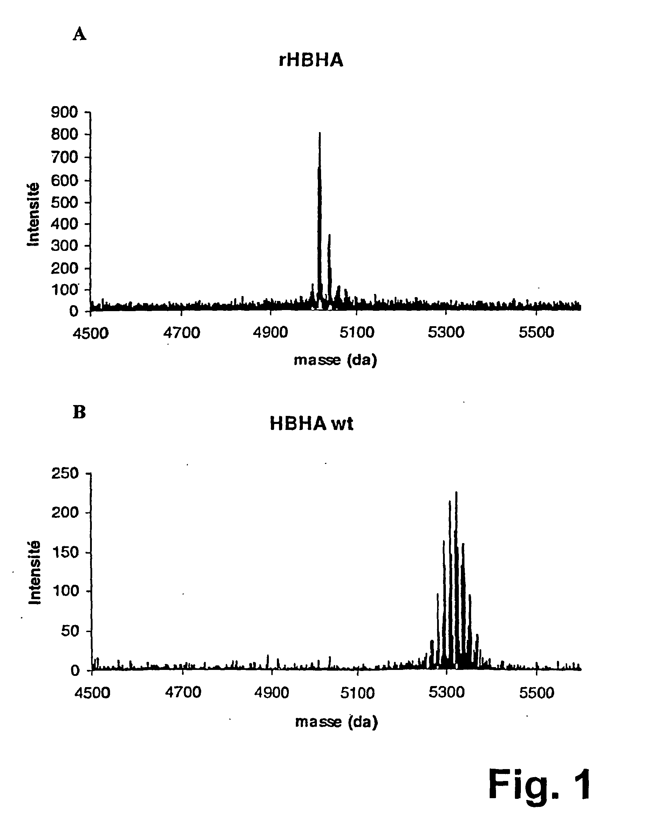

- Figure 1 : détermination de la masse du peptide correspondant au domaine de liaison à l'héparine des HBHA native et recombinante. Lesdites HBHA ont été soumises à une digestion par l'Endoprotéinase Glu-C (Endo-Glu ; EC3.4.24.33) sur la nuit. Les fragments correspondant au domaine de liaison à l'héparine ont été purifiés par HPLC. Les masses des fragments provenant de la HBHA recombinante (A) et de la HBHA native (B) ont ensuite été analysées par spectrométrie de masse.

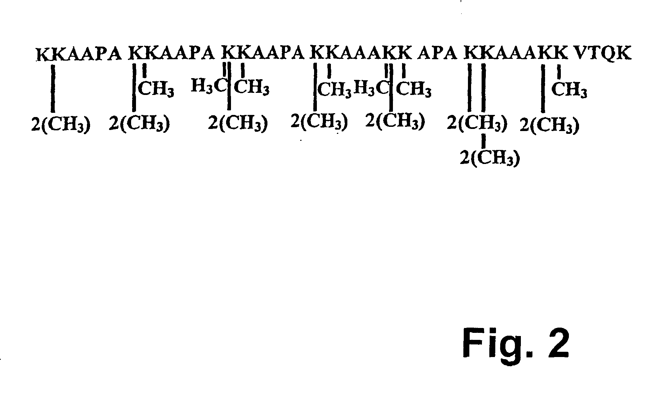

- Figure 2 : représentation du domaine de liaison à l'héparine de la HBHA produite par *M. bovis* BCG ou *M. smegmatis* (HBHA recombinante méthylée). Les lysines modifiées en mono- ou en diméthyllysines ont été identifiées selon la technique de dégradation d'Edman.

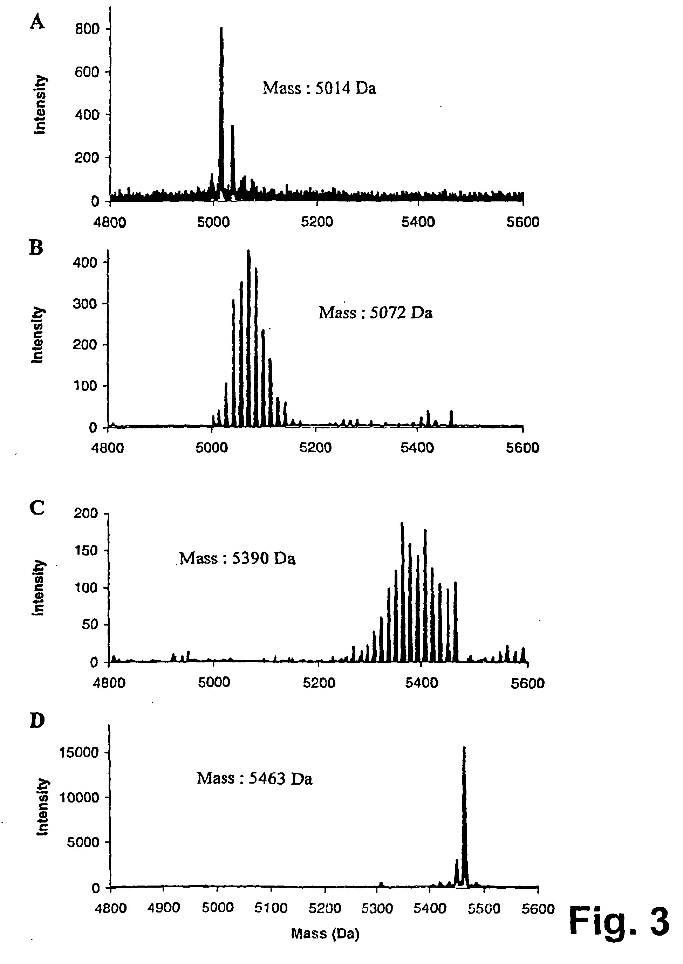

- Figure 3 : détermination de la masse du peptide correspondant au domaine de liaison à l'héparine de la HBHA recombinante non méthylée et de la HBHA recombinante méthylée par voie chimique. Les différentes formes de la HBHA ont été soumises à une digestion par l'Endo-Glu sur la nuit. Les fragments correspondant au domaine de liaison à l'héparine ont été purifiés par HPLC. Les masses des fragments provenant de la HBHA recombinante non méthylée (A), de la HBHA recombinante méthylée chimiquement pendant 6 min (B), 31 min (C) et 120 min (D) ont été analysées par spectrométrie de masse.

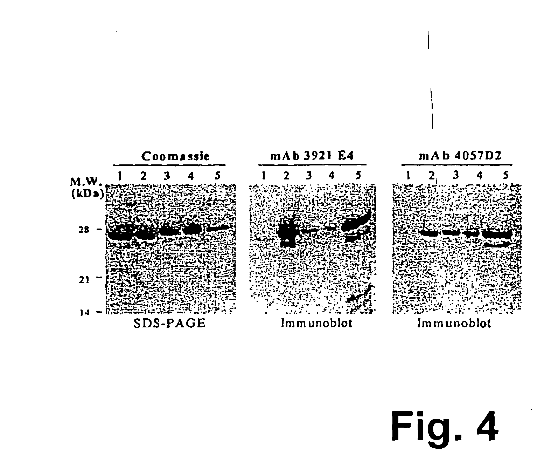

- Figure 4 : analyse en SDS-PAGE et en immunoblot de la HBHA recombinante (1), de la HBHA recombinante méthylée chimiquement pendant 6 min (2), 31 min (3), 120 min (4), et de la HBHA native (5). Les analyses en immunoblot ont été réalisées à l'aide des deux anticorps monoclonaux 3921 E4 et 4057D2 (Rouse et coll., 1991, supra).

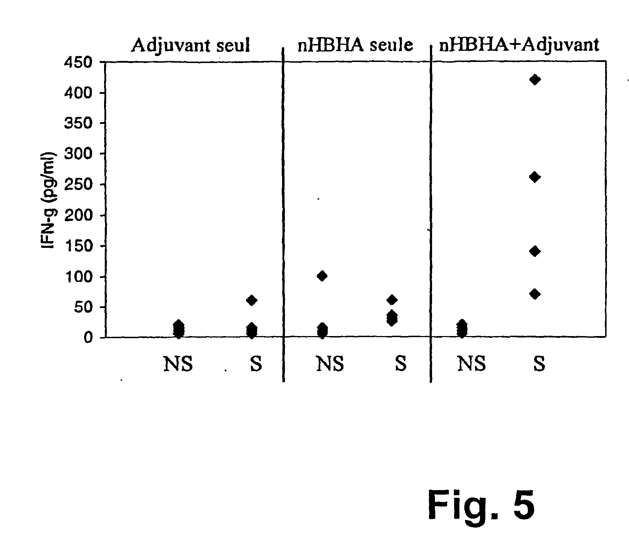

- Figure 5 : mesure de la réponse immunitaire cellulaire induite par l'injection de différentes préparations. Les cellules spléniques de quatre souris par groupe ont été mises en culture dix semaines après la première immunisation. Les cellules ont été non stimulées (NS) ou stimulées (S) pendant 72 h avec de la HBHA native (2 µg/ml). La concentration en IFN-γ a ensuite été dosée dans les surnageants de culture.

L'invention sera mieux comprise à l'aide de la description détaillée suivante, donnée à titre purement illustratif. Il est entendu que la présente invention ne se limite en aucune façon aux exemples figurant dans ladite description détaillée.

### DESCRIPTION DETAILLEE DE L'INVENTION

### I - Matériel et méthodes

### I-1- Souches bactériennes et conditions de culture

Les souches de *M. bovis* BCG 1173P2 (OMS), *M. tuberculosis* MT103 et *M. smegmatis* MC²155 ont été cultivées en milieu Sauton (Menozzi et coll., 1996, supra). La souche *E. coli* BL21 (DE3)(pET-*hbhA*) (Pethe et coll., 2000, supra) a été cultivée en milieu LB additionné de 30 µg/ml de kanamycine.

### I-2- Purification de la HBHA

Les HBHA native et recombinante ont été isolées comme décrit (Menozzi et coll., 1996, supra ; Pethe et coll., 2000, supra). L'étape finale de purification a été réalisée par HPLC en phase inverse (Beckman Gold System) à l'aide d'une colonne de type nucléosyl-C18 équilibrée dans 0,05 % d'acide trifluoroacétique. L'élution a été effectuée au moyen d'un gradient linéaire de 0 à 80 % d'acétonitrile préparé dans de l'acide trifluoroacétique à 0,05 %.

### I-3- Analyse des peptides ou protéines par spectrométrie de masse

Les échantillons (0,1 à 10 picomoles) ont été préparés par la méthode de la « goutte sèche ».

S'agissant des peptides, un volume de solution de 0,5 µl a été mélangé à de l'acide α-cyano-4-hydroxycinnaminique dissous extemporanément, à raison de 10 mg/ml dans une solution contenant 50 % de CH₃CN et 0,1 % d'acide trifluoroacétique. Après dépôt sur la plaque d'analyse, les échantillons ont été séchés. Les analyses par spectrométrie de masse ont été effectuées au moyen d'un appareil de type MALDI-TOF Voyager-DE-STR (Applied BioSystems, Foster City, CA). Les dépôts contenant des peptides de moins de 3000 Da ont été analysés à l'aide des paramètres de réglage suivants : modes positif et réflecteur, voltage d'accélération de 20 kV, tension de grille de 61 %, temporisation d'extraction de 90 ns, et seuil de masse inférieur de 500 Da. Pour les peptides dont la masse est comprise entre 3000 et 10000 Da, les paramètres de réglage sont : modes positif et réflecteur, voltage d'accélération de 25 kV, tension de grille de 65 %, temporisation d'extraction de 250 ns, et seuil de masse inférieur de 1000 Da. Les spectres ont été calibrés de manière externe à partir des ions monoisotopiques [M+H⁺] de différents peptides.

S'agissant des protéines, un échantillon de 0,5 µl a été mélangé à de l'acide sinapinique dissous extemporanément, à raison de 10 mg/ml dans une solution contenant 50 % de CH₃CN et 0,1 % d'acide trifluoroacétique. Après dépôt et séchage, les analyses par spectrométrie de masse ont été effectuées en utilisant les paramètres de réglage suivants : modes positif et linéaire, voltage d'accélération de 25 kV, tension de grille de 92 %, temporisation d'extraction de 750 ns, et seuil de masse inférieur de 1000 Da. Les spectres ont été calibrés de manière externe à partir des masses moyennes des ions [M+H⁺] de la thiorédoxine de *E. coli* et de l'apomyoglobine équine (Applied BioSystems).

### I-4- Digestion des protéines par l'Endo-Glu et séparation des peptides

1 nanomole de HBHA lyophilisée ou de HBHA recombinante purifiée par chromatographie sur héparine-sépharose suivie d'une HPLC en phase inverse, a été digérée sur la nuit en présence de 5 % d'Endo-Glu (Roche) dans 100 mM de tampon phosphate (pH 8,0). Après digestion enzymatique, les peptides résultants ont été séparés par HPLC en phase inverse à l'aide d'une colonne de type UltraSphere ODS de Beckman (2 x 200 mm) dans un gradient d'élution linéaire de 0 à 60 % d'acétonitrile préparé dans 0,1 % d'acide trifluoroacétique.

### I-5- Analyse des acides aminés et détermination des séquences

Afin d'effectuer une analyse de la composition en acides aminés complète, la HBHA native purifiée par HPLC a été hydrolysée sous chauffage constant à 110 °C dans une solution de HCl 6N pendant 14 à 16 h. La composition en acides aminés a été déterminée à l'aide d'un analyseur de type Gold System de Beckman. La séquence peptidique amino-terminale a été déterminée grâce à la méthode automatisée de dégradation d'Edman au moyen d'un appareil à liquide pulsé (Procise 492, Applied BioSystems) équipé d'un analyseur d'acides aminés 120A. Pour chaque étape de détermination de séquence, les échantillons comprenaient 10 à 20 µl, ce qui correspondait à une quantité de peptide allant de 250 à 500 picomoles.

### I-6- Méthylation chimique des résidus lysines

Le procédé de méthylation chimique des résidus lysines de la HBHA recombinante a été inspiré de la littérature (Means, 1977, supra). En substance, la HBHA recombinante purifiée sur une colonne d'héparine-sépharose a été dialysée pendant 1 h à 4 °C contre 250 volumes de tampon borate à 100 mM (pH 9,0). Après dialyse, des échantillons de 3 ml de solution de protéine à 1 mg/ml ont été transférés dans des tubes de verre fermés contenant 70 µl de solution fraîchement préparée de NaBH₄ à 40 mg/ml et 6 µl de solution de formaldéhyde à 37 % (formaline, Sigma, St. Louis). Les tubes ont été maintenus dans de la glace. Des échantillons de 200 µl ont été prélevés toutes les dix minutes afin de vérifier par immunoblotting et analyse par spectrométrie de masse la complétude de la réaction de méthylation.

### I-7- Test de méthylation enzymatique de la HBHA recombinante

100 ml de cultures de *M. smegmatis* ou *M. bovis* BCG, à une densité optique mesurée à 600 nm (DO₆₀₀) de 0,5, ont été centrifugés à 10000 x g pendant 15 min. Le culot a été resuspendu dans 10 ml de tampon Hepes à 50 mM (pH 7,4) contenant 1 mM de AEBSF (Pefabloc Sc., Roche) et 15 % (v/v) de glycérol (tampon A). Les cellules ont ensuite subi une sonication continue pendant 10 min à 4 °C à l'aide d'un sonicateur de type Branson, la puissance délivrée en sortie étant réglée sur 5. Le lysat de cellules totales a été centrifugé à 4 °C, à 20000 × g pendant 15 min. Afin de réaliser des tests de méthylation, 300 µl de lysat total clarifié à 1 mg de protéine par ml ont été mélangés avec 40 µl de [méthyl-¹⁴C]AdoMet (60 mCi/mmoi, Amersham Pharmacia Biotech), 100 µl de HBHA recombinante purifiée sur colonne héparine à 0,5 mg/ml, 5 µl de MgCl₂ à 1M et 55 µl de tampon A. Les tests de méthylation ont été effectués à 25 °C. Des échantillons de 100 µl ont été prélevés au cours du temps afin de vérifier par autoradiographie le niveau de méthylation de la HBHA recombinante.

### I-8- Animaux

Les études ont été réalisées sur des souris BALB/c femelles âgées de huit semaines (Iffa Credo, France). Pour les infections par *M. tuberculosis,* les souris ont été transférées dans un confinement de type P3.

### I-9- Immunisation

Les souris ont été immunisées trois fois à deux semaines d'intervalle en sous-cutané à la base de la queue, avec 5 µg de HBHA native par dose, émulsifiés ou non dans une solution de diméthyldioctadécylammonium (DDA, 150 µl/dose, Sigma) et de lipide A monophosphorylé (MPL, 25 µg/dose, Sigma). Au moment de la première injection, un groupe de souris a reçu une injection de BCG (souche Paris, 5.10⁵ CFU) en sous-cutané. Les souris ont été infectées dix semaines après la première immunisation.

La même expérience est conduite en remplaçant, dans les doses servant à l'immunisation, la HBHA native par (i) la HBHA recombinante non méthylée, et (ii) la HBHA recombinante méthylée.

### I-10- Infections expérimentales

Dès que la DO₆₀₀ atteignait 0,5, les cultures de *M. tuberculosis* étaient rincées une fois dans du milieu Sauton, suspendues en Sauton additionné de 30 % de glycérol, puis aliquotées pour être finalement congelées à - 80 °C. Préalablement aux infections, une aliquote était décongelée, et le nombre de CFU déterminé. Les souris ont été infectées en intraveineux dans la veine latérale de la queue par un inoculum de 10⁵ CFU de *M. tuberculosis* suspendues dans du tampon phosphate (PBS, pH 7,4), dans un volume final de 200 µl. Quatre souris par groupe ont été sacrifiées après six semaines. Le nombre de bactéries a été déterminé dans la rate, le foie et les poumons de chaque souris infectée, en étalant les dilutions des organes broyés sur du milieu 7H11.

Les organes des souris vaccinées par le BCG ont été étalés sur des boîtes 7H11 contenant 2 µg/ml d'hydrazide de l'acide 2-thiophène-carboxylique, afin d'inhiber la croissance des BCG résiduels. Les colonies ont été comptées après deux semaines d'incubation à 37 °C. L'efficacité de protection a été exprimée en log₁₀ de réduction du nombre de bactéries présentes dans les organes des souris immunisées en comparaison avec l'énumération relative au groupe qui avait reçu l'adjuvant seul. Les résultats ont été obtenus à partir de groupes de quatre souris.

### I-11- Cultures de lymphocytes et dosage de l'IFN-γ

Les lymphocytes des rates ont été purifiés comme décrit (Andersen et coll. 1991. Infect. Immun. 59 : 1558-1563). Les lymphocytes de quatre souris par expérience ont été mis en culture dans des plaques de 96 puits (NUNC) contenant 2.10⁵ cellules/puits, dans 200 µl de RPMI 1640 (Gibco, France) supplémenté par 50 µM de 2-mercaptoéthanol (Merck, Allemagne), 50 µg/ml de pénicilline-streptomycine (Gibco), 1 mM de glutamax (Gibco) et 10 % de sérum de veau foetal (Roche).

La concanavaline A à 5 µg/ml a été utilisée comme témoin positif de viabilité cellulaire. La HBHA native a été utilisée à une concentration finale de 5 µg/ml. Les surnageants ont été récupérés 72 heures après le début de la stimulation afin de doser l'IFN-γ. L'IFN-γ a été détecté par un test ELISA de type sandwich. Les anticorps monoclonaux anti-IFN-γ utilisés ont été obtenus à partir des clones R4-6A2 (Pharmingen, USA) pour la capture, et SMG1-2 (Pharmingen) pour la détection.

### II - Résultats et exemples

### II-1- Caractérisation de la modification post-traductionnelle de la HBHA native

L'analyse par spectrométrie de masse a révélé que la HBHA recombinante avait un poids moléculaire (PM) de 21340, ce qui correspondait au PM déduit de la séquence nucléotidique codant la HBHA mycobactérienne (gène *hbhA* ou encore Rv0475 chez *M. tuberculosis* H37Rv) (Menozzi et coll., 1998, supra). En revanche, le PM de la HBHA native était de 21610, soit un PM de 270 supérieur à celui de la HBHA recombinante. En conséquence, la HBHA produite par les mycobactéries subissait une modification, laquelle n'était pas retrouvée au niveau de la protéine recombinante produite par *E. coli.* Afin de définir la nature exacte de cette modification, les HBHA native et recombinante ont été soumises à une hydrolyse par l'Endo-Glu, et la masse des peptides obtenus a été déterminée par spectrométrie de masse. La seule différence entre les HBHA native et recombinante a été identifiée au niveau du domaine carboxy-terminal desdites protéines. La masse de ce domaine était de 4342 Da pour la HBHA native, et de seulement 4076 Da pour la HBHA recombinante. Cette différence d'environ 270 Da correspondait à la différence de masse mesurée entre les protéines HBHA entières. Aussi, la ou les modifications post-traductionnelles de la HBHA native ont pu être localisées dans le domaine C-terminal. De plus, le spectre de masse correspondant au dit domaine était constitué d'un seul pic pour la HBHA recombinante, alors qu'il comptait cinq pics pour la HBHA native, ces pics étant séparés les uns des autres par 14 Da (Figure 1).

### II-2- Détermination de la modification post-traductionnelle de la HBHA native

En vue de l'identification précise des acides aminés modifiés, la séquence du domaine de liaison à l'héparine a été déterminée par la méthode de dégradation d'Edman selon les procédures classiques. Cette étude a révélé que seules les lysines étaient modifiées. En outre, sur les quinze résidus lysines présents dans le domaine C-terminal de la HBHA, seuls deux présentaient le temps de rétention du standard lysine. Les treize autres résidus avaient des temps de rétention correspondant aux standards glutamine et/ou arginine. Dans un premier temps, puisque (i) l'analyse par spectrométrie de masse avait révélé qu'il y avait un incrément de 14 Da entre les différents fragments de la HBHA native, et (ii) seules les lysines étaient modifiées, l'hypothèse avait été émise que les lysines du domaine C-terminal pouvaient être méthylées, donnant des mono-, des di-, ou encore des triméthyllysines. Cette hypothèse ne s'est cependant révélée qu'en partie exacte, dans la mesure où aucune triméthyllysine n'a en définitive été identifiée dans la HBHA native. Cette vérification a été effectuée au moyen de standards de calibrage correspondant à des mono-, di- et triméthyllysines, respectivement. Les lysines modifiées avaient des temps de rétention conformes à ceux de la mono- et de la diméthyllysine, mais pas à celui de la triméthyllysine (Figure 2).

Une analyse des acides aminés, en incluant la mono-, la di- et la triméthyllysine à titre de standards, a confirmé ce résultat.

### II-3- Méthylation chimique de la HBHA recombinante

La HBHA recombinante a été méthylée chimiquement et ensuite soumise à l'analyse par spectrométrie de masse. Comme indiqué sur la figure 3, la masse du peptide correspondant au domaine C-terminal de la HBHA recombinante augmentait au fur et à mesure que la méthylation chimique progressait.

En outre, le degré de méthylation influençait la réactivité des peptides avec les anticorps monoclonaux 3921 E4 et 4057D2 (Rouse et coll., 1991, supra) (Figure 4). Comme précédemment décrit (Menozzi et coll., 1998, supra), la HBHA recombinante n'était pas reconnue par l'anticorps 4057D2, alors qu'elle l'était faiblement par l'anticorps 3921 E4. En revanche, comme indiqué sur la figure 4, le degré de méthylation de la HBHA recombinante affectait différemment son affinité pour ces deux anticorps, montrant que la méthylation d'une protéine pouvait jouer un rôle important dans son antigénicité.

### II-4- Méthylation enzymatique de la HBHA recombinante

Afin de déterminer si la méthylation des lysines de la HBHA native était le fait d'une activité enzymatique, un test de méthylation *in vitro* spécifique de la HBHA recombinante a été mis au point à partir d'un lysat mycobactérien. Des cultures mycobactériennes ont été lysées par sonication. Les lysats totaux, de même que des fractions cytoplasmiques et pariétales, ont été utilisés comme sources enzymatiques pour tenter de transférer des groupements [¹⁴C]méthyles du donneur [1⁴C-méthyl]AdoMet vers l'accepteur représenté par la HBHA recombinante. L'incubation des lysats totaux de *M. tuberculosis, M. bovis* BCG et *M. smegmatis,* contenant de la [¹⁴C-méthyl]AdoMet, avec la HBHA recombinante a résulté en l'incorporation de groupements [¹⁴C]méthyles dans ladite HBHA (Figure 2). En revanche, lorsque les lysats étaient chauffés à 95 °C, ils n'étaient plus capables de catalyser la réaction de transméthylation. Aussi, la ou les méthyltransférases mycobactériennes responsables de la méthylation de la HBHA étaient thermosensibles.

L'isolement de la ou des méthyltranférases est envisagé selon différentes approches.

Dans un premier cas, les protéines présentes dans un lysat mycobactérien sont séparées par chromatographie échangeuse d'ions, HPLC ou d'affinité, en suivant les fractions capables de catalyser la réaction de transméthylation à partir de la [¹⁴C-méthyl]AdoMet sur la HBHA recombinante. De tels enrichissements sont poursuivis jusqu'à obtenir un échantillon dans lequel la ou les méthyltransférases sont suffisamment pures pour en déterminer la séquence. Ainsi, en se référant à la séquence connue du génome de *M. tuberculosis* H37Rv (Cole et coll., 1998, supra), le ou les gènes codant la ou les méthyltranférases sont identifiés, puis clonés suivant les techniques connues de l'homme du métier.

Une seconde approche consistait à rechercher dans le génome de *M. tuberculosis* H37Rv les gènes candidats codant potentiellement des méthyltranférases sur la base d'une homologie de séquence avec la séquence de gènes de méthyltranférases connus et identifiés en tant que tels dans les banques de données. Ainsi, ont été retenus cinq gènes candidats, à savoir Rv0208c, Rv0380, Rv1405, Rv1644 et Rv3579. Ces gènes sont clonés et exprimés chez *E. coli.* Les produits desdits gènes sont ensuite purifiés et testés pour leur capacité à méthyler la HBHA recombinante à partir du donneur de méthyle AdoMet marquée radioactivement.

### II-5- Production de la HBHA par M. smegmatis

Il a été démontré que *M. smegmatis* n'exprimait pas la HBHA (Pethe et coll., 2001, supra). Pourtant, il était possible de transférer des groupements [¹⁴C]méthyles à partir de [¹⁴C-méthyl]AdoMet sur la HBHA recombinante en utilisant un lysat de ce microorganisme (Figure 2). Aussi était-il suggéré que *M. smegmatis* possédait la machinerie enzymatique responsable de la réaction de transméthylation de la HBHA. Dans le but de vérifier cette hypothèse, la souche *M. smegmatis* MC²155 a été transformée avec un dérivé du plasmide pRR3 contenant le gène *hbhA* (Rv0475) codant la HBHA chez *M. bovis* BCG, afin d'obtenir la souche *M. smegmatis*(*pRR-hbhA*). La production de HBHA a été analysée par western blot. La HBHA produite par *M. smegmatis*(*pRR-hbhA*), appelée MS-HBHA, était reconnue par les anticorps monoclonaux 3921 E4 et 4057D2, suggérant fortement que ladite MS-HBHA était modifiée post-traductionnellement à l'instar de la HBHA native de *M. bovis* BCG. La MS-HBHA a été purifiée et soumise à une hydrolyse par l'Endo-Glu. L'analyse par spectrométrie de masse des produits de digestion ainsi obtenus, ainsi que la détermination de la séquence peptidique du domaine C-terminal de la MS-HBHA, ont montré qu'elle présentait effectivement le même type de modification post-traductionnelle que la HBHA de *M. bovis.* En conséquence, *M. smegmatis* possédait une machinerie enzymatique capable de catalyser la méthylation de la HBHA recombinante.

En conséquence, pour réaliser les expériences de vaccination, la HBHA native est alternativement purifiée à partir de la souche transformée *M. smegmatis(pRR-hbhA).*

### II-6- Etude de la HBHA native comme antigène protecteur

La réponse immunitaire engendrée par la HBHA native, de même que son pouvoir protecteur contre une infection par *M. tuberculosis,* ont été testés dans le modèle murin.

Ces expériences sont également réalisées en utilisant la HBHA recombinante, sous ses formes non méthylée et méthylée.

Le protocole d'immunisation a été inspiré de la littérature (Brandt et coll. 2000. Infect. Immun. 68 : 791-795). Les adjuvants DDA et MPL ont été respectivement utilisés à raison de 150 µg et 25 µg par dose.

Le groupe 1 a été vacciné avec l'adjuvant seul contenu dans 200 µl de tampon PBS. Le groupe 2 a été vacciné avec 5 µg de HBHA native purifiée et émulsionnée dans 200 µl de mélange PBS-adjuvant. Le groupe 3 a été vacciné avec 5 µg de HBHA native seule, en solution dans 200 µl de PBS. Les souris ont reçu trois injections des différentes préparations à deux semaines d'intervalle. Un quatrième groupe (témoin positif) a été vacciné avec une dose de 5.10⁵ CFU de BCG.

Du sang a été prélevé sur l'ensemble des souris des différents groupes, dix jours après la dernière injection des préparations vaccinales, afin de tester la production d'anticorps spécifiques de la HBHA native. Pour chaque groupe, les dosages en IgG ont été réalisés sur des mélanges de sérums. Le titre en anticorps a été défini comme correspondant à la dilution maximale des sérums donnant une valeur trois fois supérieure au blanc. Le tableau 1 suivant fournit une mesure des titres en IgG induits par l'injection des différentes préparations.

**Tableau 1**

| | Groupe 1 | Groupe 2 | Groupe 3 | Groupe 4 |
|---|---|---|---|---|
| | Adjuvant | HBHA+adjuvant | HBHA | BCG |
| IgG totales | < 10 | 73000 | 5000 | < 50 |
| IgG1 | < 10 | 580000 | 24000 | < 10 |
| IgG2a | < 10 | 17000 | 800 | < 20 |
| IgG2b | < 10 | 8500 | 90 | < 10 |
| IgG3 | < 10 | 750 | 150 | < 10 |

Les résultats ont montré que les souris vaccinées avec la HBHA (groupes 2 et 3) produisaient des taux importants d'IgG1, et produisaient également des IgG2a, IgG2b, ainsi que des IgG3. Ces types d'anticorps reflétaient la génération d'une réponse mixte TH1/TH2. La présence de l'adjuvant (groupe 2) ne modifiait pas le profil de la réponse par rapport à la protéine HBHA seule (groupe 3). Toutefois, ledit adjuvant permettait de produire environ 10 fois plus des différentes IgG (Tableau 1).

Afin de tester la réponse cellulaire, quatre souris par groupe ont été sacrifiées dix semaines après la première injection. Les lymphocytes ont été récupérés et stimulés *in vitro* par la HBHA native. Après stimulation, la production d'IFN-γ a été testée. Comme indiqué sur la figure 5, seuls les lymphocytes purifiés à partir des souris du groupe 2, vaccinées avec la HBHA native associée à l'adjuvant, produisaient de l'IFN-γ spécifique de ladite HBHA.

Enfin, des expériences ont été effectuées dans le but de tester le pouvoir protecteur de la HBHA native contre une infection par *M. tuberculosis.* Le groupe 3, vacciné par la HBHA seule, a été écarté au profit du groupe 2 étant donné que la réponse immunitaire, tant humorale (Tableau 1) que cellulaire (Figure 5), y était apparue de meilleure qualité à la lumière des résultats expérimentaux. Dix semaines après la première injection de préparations vaccinales, les souris ont été infectées par voie intraveineuse avec 10⁵ CFU de *M. tuberculosis.* Quatre souris par groupe ont été sacrifiées six semaines après l'infection afin d'énumérer le nombre de CFU présentes dans les différents organes des souris. La charge bactérienne a été déterminée dans le foie, la rate et les poumons des animaux. La résistance a été définie comme la différence de charge bactérienne, exprimée en log₁₀, entre le groupe contrôle 1, vacciné par l'adjuvant seul, et les groupes 2 et 4, respectivement vaccinés par la HBHA associée à l'adjuvant et par le BCG. Le tableau 2 suivant indique l'efficacité de la protection induite par les différentes immunisations.

**Tableau 2**

| | Foie | | Rate | | Poumons | |
|---|---|---|---|---|---|---|
| | CFU (log₁₀) | Résistance | CFU (log₁₀) | Résistance | CFU (log₁₀) | Résistance |
| Groupe 1 Adjuvant | 5,60±0,20 | | 5,85±0,21 | | 5,27±0,25 | |
| Groupe 2 HBHA+ adjuvant | 4,66±0,35 | 0,94 | 5,00±0,04 | 0,85 | 4,34±0,17 | 0,93 |
| Groupe 4 BCG | 4,41±0,20 | 1,19 | 4,68±0,25 | 1,17 | 4,45±0,20 | 0,82 |

L'énumération des CFU a montré que la réponse immunitaire engendrée par la HBHA native était capable de rendre la souris partiellement résistante à une infection par *M. tuberculosis.* La résistance observée était d'ailleurs du même ordre de grandeur, qu'il s'agisse de la HBHA native ou du vaccin de référence de l'état de la technique, à savoir le BCG. En conséquence, des injections de HBHA native protégeaient la souris d'une infection par *M. tuberculosis,* dans des proportions voisines de celles du vaccin BCG.

Cette expérience est conduite en utilisant les formes méthylée et non méthylée de la HBHA recombinante, afin de comparer le niveau et l'efficacité de la protection induite à ceux observés avec la HBHA native. Ainsi, la HBHA recombinante méthylée, en ce qu'elle est immunogène, engendre, chez l'animal, une résistance à une infection par *M. tuberculosis* tout aussi efficace que celle induite par la HBHA native.

Aussi, l'un des objets de la présente invention est un vaccin sous-unitaire destiné au traitement des infections mycobactériennes et comprenant, dans sa formulation, la HBHA native.

Dans le cadre de la production de compositions vaccinales à l'échelle industrielle, il est stratégique d'utiliser des organismes producteurs génétiquement recombinants, souvent plus intéressants que les organismes producteurs sauvages en ce que les premiers sont faciles à transformer par les séquences nucléotidiques des seconds, codant la ou les protéines d'intérêt, et en ce qu'ils sont judicieusement choisis, notamment pour leur innocuité et leurs paramètres de croissance aisément contrôlables, tels en particulier qu'il n'est pas nécessaire d'investir dans du matériel spécifique coûteux. C'est pourquoi, un objet préféré de l'invention concerne un vaccin sous-unitaire destiné au traitement des infections mycobactériennes avantageusement caractérisé en ce qu'il comprend, dans sa formulation, la HBHA méthylée dans sa version recombinante, c'est-à-dire produite par un hôte cellulaire recombinant méticuleusement choisi pour répondre aux exigences industrielles et sanitaires.

## Revendications

1. Peptide recombinant immunogène, **caractérisé en ce qu'**il est une forme méthylée, du produit d'expression d'une séquence nucléotidique codant un antigène mycobactérien de type hémagglutinine de liaison à l'héparine (HBHA), au niveau du domaine de liaison à l'héparine de la HBHA de séquence KKAAPAKKAAPAKKAAPAKKAAAKKAPAKKAAAKKVTQK,

2. Peptide recombinant immunogène selon la revendication 1, **caractérisé en ce que** l'antigène mycobactérien de type hémagglutinine de liaison à l'héparine est obtenu à partir de *M. bovis* BCG ou *M. tuberculosis.*

3. Peptide recombinant immunogène selon la revendication 1 ou 2, **caractérisée en ce que** la séquence nucléotidique code pour une partie de la protéine HBHA contenant au moins le domaine de liaison à l'héparine de la HBHA.

4. Peptide recombinant immunogène selon l'une quelconque des revendications 1 à 3, **caractérisé en ce que** les groupements méthyles sont portés par des résidus lysines situés dans le domaine de liaison à l'héparine de la HBHA.

5. Peptide recombinant immunogène selon la revendication 4, **caractérisé en ce que** les résidus lysines sont mono- ou diméthylés.

6. Peptide recombinant immunogène selon la revendication 4 ou 5, **caractérisé en ce que** les groupements méthyles sont portés par tout ou partie des résidus lysines situés dans le domaine de liaison à l'héparine de la HBHA.

7. Peptide recombinant immunogène selon l'une quelconque des revendications 4 à 6, **caractérisé en ce que** les groupements méthyles sont portés par tous les résidus lysines situés dans le domaine de liaison à l'héparine de la HBHA.

8. Procédé d'obtention d'un peptide recombinant immunogène selon l'une quelconque des revendications 1 à 7, **caractérisé en ce qu'**il comprend au moins les étapes suivantes :
(a) la production par un hôte cellulaire recombinant, de la protéine HBHA recombinante ;
(b) la purification de ladite protéine ; et
(c) sa méthylation post-traductionnelle ;
l'ordre des deux dernières étapes pouvant être inversé.

9. Procédé d'obtention selon la revendication 8, **caractérisé en ce que** la protéine HBHA recombinante est constituée de son domaine de liaison à l'héparine.

10. Procédé d'obtention selon la revendication 8 ou 9, **caractérisé en ce que** l'hôte cellulaire recombinant est une bactérie.

11. Procédé d'obtention selon la revendication 10, **caractérisé en ce que** la bactérie est *M. smegmatis.*

12. Procédé d'obtention selon l'une quelconque des revendications 8 à 11, **caractérisé en ce que** l'étape de méthylation est réalisée par voie chimique.

13. Procédé d'obtention selon l'une quelconque des revendications 8 à 11, **caractérisé en ce que** l'étape de méthylation est réalisée par voie enzymatique.

14. Procédé d'obtention selon la revendication 13, **caractérisé en ce que** la réaction de méthylation est catalysée par au moins une méthyltranférase contenue dans les extraits de protéines totales de mycobactéries.

15. Procédé d'obtention selon la revendication 14, **caractérisé en ce que** la au moins une méthyltranférase est contenue dans les extraits de protéines totales de *M. bovis* BCG ou *M. smegmatis.*

16. Peptides recombinants immunogènes méthylés susceptibles d'être obtenus par un procédé selon l'une quelconque des revendications 8 à 15.

17. Utilisation d'une forme méthylée de la HBHA native, ou d'une forme méthylée de la HBHA recombinante au niveau du domaine de liaison à l'héparine de séquence KKAAPAKKAAPAKKAAPAKKAAAKKAPAKKAAAKKVTQK, pour la préparation de vaccins contre des infections mycobactériennes.

18. Utilisation selon la revendication 17, pour la préparation de vaccins contre des infections à *M. bovis* ou *M. tuberculosis.*

19. Utilisation selon la revendication 17 ou 18, **caractérisée en ce que** la forme recombinante est un peptide recombinant immunogène selon l'une quelconque des revendications 1 à 7.

20. Composition immunogène **caractérisée en ce qu'**elle comprend, en tant que principe actif, une forme méthylée de la HBHA native, ou une forme méthylée de la HBHA recombinante au niveau du domaine de liaison à l'héparine de séquence KKAAPAKKAAPAKKAAPAKKAAAKKAPAKKAAAKKVTQK, et au moins un excipient pharmaceutiquement compatible.

21. Composition immunogène selon la revendication 20, **caractérisée en ce que** la forme méthylée est associée à un ou plusieurs adjuvants.

22. Composition immunogène selon la revendication 20 ou 21, **caractérisée en ce qu'**elle comprend en outre des excipients pharmaceutiquement compatibles, tels que l'eau, un tampon salin, du dextrose, du glycérol, de l'éthanol, ou des mélanges de ceux-ci.

23. Composition immunogène selon l'une quelconque des revendications 20 à 22, **caractérisée en ce que** la forme méthyle est la HBHA native.

24. Composition immunogène selon l'une quelconque des revendications 20 à 22, **caractérisée en ce que** la forme méthylée est un peptide recombinant selon l'une quelconque des revendications 1 à 7.

25. Composition immunogène selon l'une quelconque des revendications 20 à 24, formulée pour une administration par voie nasale, orale, sous-cutanée, intradermique, intramusculaire, vaginale, rectale, oculaire, ou auriculaire.

26. Composition immunogène selon la revendication 25 comprenant des composés auxiliaires choisi parmi des agents mouillants, émulsifiants ou tampons.

27. Composition immunogène selon l'une quelconque des revendications 20 à 26, **caractérisée en ce qu'**elle comprend entre 0,1 et 20 µg de protéine HBHA purifiée par dose.

28. Composition immunogène selon la revendication 27, **caractérisée en ce qu'**elle comprend 5 µg de protéine HBHA purifiée par dose.

## Claims

1. An immunogenic recombinant peptide, **characterized in that** it is a methylated form of an expression product of a nucleotide sequence coding for a heparin-binding hemagglutinin (HBHA) type mycobacterial antigen, at the heparin-binding region of the HBHA with sequence KKAAPAKKAAPAKKAAPAKKAAAKKAPAKKAAAKKVTQK.

2. An immunogenic recombinant peptide according to claim 1, **characterized in that** the heparin-binding hemagglutinin type mycobacterial antigen is obtained from *M. bovis* BCG or *M tuberculosis,*

3. An immunogenic recombinant peptide according to claim 1 or 2, **characterized in that** the nucleotide sequence codes for a portion of the HBHA protein containing at least the heparin-binding region of the HBHA.

4. An immunogenic recombinant peptide according to any one of claims 1 to 3, **characterized in that** the methyl groups are carried by lysine residues located in the heparin-binding region of the HBHA.

5. An immunogenic recombinant peptide according to claim 4, **characterized in that** the lysine residues are mono- or di-methylated.

6. An immunogenic recombinant peptide according to claim 4 or claim 5, **characterized in that** the methyl groups are carried by all or a portion of the lysine residues located in the heparin-binding region of the HBHA.

7. An immunogenic recombinant peptide according to any one of claims 4 to 6, **characterized in that** the methyl groups are carried by all of the lysine residues located in the heparin-binding region of the HBHA.

8. A method for producing an immunogenic recombinant peptide according to any one of claims 1 to 7, **characterized in that** it comprises et least the following steps:
(a) the production of recombinant HBHA protein using a recombinant host cell;
(b) purification of said protein; and
(c) post-translational methylation thereof;
the order of the last two steps optionally being reversed.

9. A production method according to claim 8, **characterized in that** the recombinant HBHA protein is constituted by its heparin-binding region.

10. A production method according to claim 8 or 9, **characterized in that** the recombinant host cell is a bacterium.

11. A production method according to claim 10, **characterized in that** the bacterium is *M. smegmatis.*

12. A production method according to any one of claims 8 to 11, **characterized in that** the methylation step is carried out chemically.

13. A production method according to any one of claims 8 to 11, **characterized in that** the methylation step is carried out enzymatically.

14. A production method according to claim 13, **characterized in that** the methylation reaction is catalyzed by at least one methyltransferase contained in extracts from total mycobacterial proteins.

15. A production method according to claim 14, **characterized in that** the at least one methyltransferase is contained in extracts from total proteins of *M. bovis* BCG or *M smegmatis.*

16. Methylated immunogenic recombinant peptides that can be obtained using a method according to any one of claims 8 to 15.

17. Use of a methylated form of native HBHA or of a methylated form of recombinant HBHA, at the heparin-binding region with sequence KKAAPAKKAAPAKKAAPAKKAAAKKAPAKKAAAKKVTQK, for the preparation of vaccines against mycobacterial infections.

18. Use according to claim 17, to prepare vaccines against infections with *M. bovis* or *M. tuberculosis.*

19. Use according to claim 17 or 18, **characterized in that** the recombinant form is an immunogenic recombinant peptide according to any one of claims 1 to 7.

20. An immunogenic composition, **characterized in that** it comprises, as an active principle, a methylated form of native HBHA or a methylated form of recombinant HRHA, methylated at the heparin-binding region with sequence KKAAPAKKAAPAKKAAPAKKAAAKKAPAKKAAAKKVTQK, and at least one pharmaceutically compatible excipient.

21. An immunogenic composition according to claim 20, **characterized in that** the methylated form is associated with one or more adjuvants.

22. An immunogenic composition according to claim 20 or 21, **characterized in that** it further comprises pharmaceutically compatible excipients such as water, a saline buffer, dextrose, glycerol, ethanol or mixtures thereof.

23. An immunogenic composition according to any one of claims 20 to 22, **characterized in that** the methylated form is native HBHA.

24. An immunogenic composition according to any one of claims 20 to 22, **characterized in that** the methylated form is a recombinant peptide according to any one of claims 1 to 7.

25. An immunogenic composition according to any one of claims 20 to 24, formulated for nasal, oral, subcutaneous, intradermal, intramuscular, vaginal, rectal, ocular or auricular administration.

26. An immunogenic composition according to claim 25, comprising auxiliary compounds selected from wetting agents, emulsifiers or buffers.

27. An immunogenic composition according to any one of claims 20 to 26, **characterized in that** it comprises between 0.1 and 20 µg of purified HBHA protein per dose.

28. An immunogenic composition according to claim 27, **characterized in that** it comprises 5 µg of purified HBHA protein per dose.

## Patentansprüche

1. Immunogenes rekombinantes Peptid, **dadurch gekennzeichnet, dass** es eine methylierte Form des Expressionsprodukts einer ein mycobaktierielles Antigen vom Typ Heparin-bindendes Hämagglutinin (HBHA) kodierenden Nukleotidsequenz im Bereich der Heparin-Bindungsdomäne des HBHA mit der Sequenz KKAAPAKKAAPAKKAAPAKKAAAKKAPAKKAAAKKVTQK ist.

2. Immunogenes rekombinantes Peptid nach Anspruch 1, **dadurch gekennzeichnet, dass** das mycobakterielle Antigen vom Typ Heparin-bindendes Hämagglutinin mittels *M. bovis* BCG oder *M. tuberculosis* erhalten wird.

3. Immunogenes rekombinantes Peptid nach Anspruch 1 oder 2, **dadurch gekennzeichnet, dass** die Nukleotidsequenz für einen Teil des HBHA-Proteins kodiert, das wenigstens die Heparin-Bindungsdomäne des HBHA enthält.

4. Immunogenes rekombinantes Peptid nach einem beliebigen der Ansprüche 1 bis 3, **dadurch gekennzeichnet, dass** die Methylgruppen von in der Heparin-Bindungsdomäne des HBHA liegenden Lysin-Resten getragen sind.

5. Immunogenes rekombinantes Peptid nach Anspruch 4, **dadurch gekennzeichnet, dass** die Lysin-Reste mono- oder dimethyliert sind.

6. Immunogenes rekombinantes Peptid nach Anspruch 4 oder 5, **dadurch gekennzeichnet, dass** die Methylgruppen von allen oder von Teilen der in der Heparin-Bindungsdomäne des HBHA liegenden Lysin-Reste getragen sind.

7. Immunogenes rekombinantes Peptid nach einem beliebigen der Ansprüche 4 bis 6, **dadurch gekennzeichnet, dass** die Methylgruppen von allen in der Heparin-Bindungsdomäne des HBHA liegenden Lysin-Resten getragen sind.

8. Verfahren zum Herstellen eines immunogenen rekombinanten Peptids gemäß einem beliebigen der Ansprüche 1 bis 7, **dadurch gekennzeichnet, dass** es wenigstens die folgenden Schritte umfasst:
(a) Produktion des rekombinanten HBHA-Proteins durch einen rekombinanten Zellwirt;
(b) Reinigung des Proteins; und
(c) seine posttranslatorische Methylierung;
wobei die Reihenfolge der beiden letzten Schritte vertauscht werden kann.

9. Herstellungsverfahren nach Anspruch 8, **dadurch gekennzeichnet, dass** das rekombinante HBHA-Protein von seiner Heparin-Bindungsdomäne gebildet ist.

10. Herstellungsverfahren nach Anspruch 8 oder 9, **dadurch gekennzeichnet, dass** der rekombinante Zellwirt ein Bakterium ist.

11. Herstellungsverfahren nach Anspruch 10, **dadurch gekennzeichnet, dass** das Bakterium *M. smegmatis* ist.

12. Herstellungsverfahren nach einem beliebigen der Ansprüche 8 bis 11, **dadurch gekennzeichnet, dass** der Methylierungsschritt chemisch erfolgt.

13. Herstellungsverfahren nach einem beliebigen der Ansprüche 8 bis 11, **dadurch gekennzeichnet, dass** der Methylierungsschritt enzymatisch erfolgt.

14. Herstellungsverfahren nach Anspruch 13, **dadurch gekennzeichnet, dass** die Methylierungsreaktion durch wenigstens eine in den Extrakten der gesamten Mycobakterienproteine enthaltene Methyltransferase katalysiert wird.

15. Herstellungsverfahren nach Anspruch 14, **dadurch gekennzeichnet, dass** wenigstens eine Methyltransferase in den Extrakten der gesamten Proteine von *M. bovis* BCG oder *M. smegmatis* enthalten ist.

16. Methylierte immunogene rekombinante Peptide, die durch ein Verfahren gemäß einem beliebigen der Ansprüche 8 bis 15 erhältlich ist.

17. Verwendung einer methylierten Form von nativem HBHA oder einer methylierten Form von rekombinantem HBHA im Bereich der Heparin-Bindungsdomäne mit der Sequenz KKAAPAKKAAPAKKAAPAKKAAAKKAPAKKAAAKKVTQK zur Gewinnung von Impfstoffen gegen mycobakterielle Infektionen.

18. Verwendung nach Anspruch 17 zur Gewinnung von Impfstoffen gegen Infektionen mit *M. bovis* oder *M. tuberculosis.*

19. Verwendung nach Anspruch 17 oder 18, **dadurch gekennzeichnet, dass** die rekombinante Form ein immunogenes rekombinantes Peptid gemäß einem beliebigen der Ansprüche 1 bis 7 ist.

20. Immunogene Zusammensetzung, **dadurch gekennzeichnet, dass** sie als Wirkstoff eine methylierte Form des nativen HBHA oder ein methylierte Form des rekombinanten HBHA im Bereich der Heparin-Bindungsdomäne mit der Sequenz KKAAPAKKAAPAKKAAPAKKAAAKKAPAKKAAAKKVTQK und wenigstens einen pharmazeutisch kompatiblen Trägerstoff umfasst.

21. Immunogene Zusammensetzung nach Anspruch 20, **dadurch gekennzeichnet, dass** die methylierte Form mit einem oder mehreren Adjuvantien assoziiert ist.

22. Immunogene Zusammensetzung nach Anspruch 20 oder 21, **dadurch gekennzeichnet, dass** sie ferner pharmazeutisch kompatible Trägerstoffe umfasst, wie Wasser, einen Salzpuffer, Dextrose, Glyzerin, Ethanol oder Mischungen aus diesen.

23. Immunogene Zusammensetzung nach einem beliebigen der Ansprüche 20 bis 22, **dadurch gekennzeichnet, dass** die methylierte Form natives HBHA ist.

24. Immunogene Zusammensetzung nach einem beliebigen der Ansprüche 20 bis 22, **dadurch gekennzeichnet, dass** die methylierte Form ein rekombinantes Peptid gemäß einem beliebigen der Ansprüche 1 bis 7 ist.

25. Immunogene Zusammensetzung nach einem der Ansprüche 20 bis 24, formuliert für eine nasale, orale, subkutane, intradermale, intramuskuläre, vaginale, rektale, okuläre oder aurikulare Verabreichung.

26. Immunogene Zusammensetzung nach Anspruch 25, umfassend Hilfsverbindungen, die aus Netzmitteln, Emulgiermitteln oder Puffermitteln gewählt sind.

27. Immunogene Zusammensetzung nach einem beliebigen der Ansprüche 20 bis 26, **dadurch gekennzeichnet, dass** sie 0,1 bis 20 µg gereinigtes HBHA-Protein pro Dosis umfasst.

28. Immunogene Zusammensetzung nach Anspruch 27, **dadurch gekennzeichnet, dass** sie 5 pµ gereinigtes HBHA-Protein pro Dosis umfasst.
